# EUROPEAN PATENT SPECIFICATION

(11) **EP 2 548 637 B1**
(45) Date of publication and mention of the grant of the patent: **20.04.2016**
(21) Application number: 10847994.0
(22) Date of filing: 07.12.2010
(51) Int. Cl.: B01J 2/12, A23P 20/00, A61J 3/06, B01J 2/00, B05D 7/00, A23G 3/34, A23G 3/26, B05C 5/02, B05D 1/02

(54) **COATING DEVICE AND COATING METHOD**
BESCHICHTUNGSVORRICHTUNG UND BESCHICHTUNGSVERFAHREN
DISPOSITIF DE REVÊTEMENT ET PROCÉDÉ DE REVÊTEMENT

(30) Priority: 19.03.2010 JP 2010064697
(43) Date of publication of application: 23.01.2013
(73) Proprietor: Kabushiki Kaisha Powrex, Itami-shi Hyogo 664-0831 (JP)
(72) Inventor: UMEMOTO Kenji, Itami-shi Hyogo 664-0831 (JP); HOSONO Tetsuya, Itami-shi Hyogo 664-0831 (JP); ENDOU Tarou, Itami-shi Hyogo 664-0831 (JP); KAMADA Hitoshi, Itami-shi Hyogo 664-0831 (JP); AKIMOTO Nanao, Itami-shi Hyogo 664-0831 (JP); HASEGAWA Koji, Itami-shi Hyogo 664-0831 (JP)
(74) Representative: Eder, Thomas
(86) International application number: PCT/JP2010/071859
(87) International publication number: WO 2011/114588

(56) References cited:
- EP-A2- 0 391 530
- GB-A- 2 418 016
- JP-A- 4 222 628
- JP-A- 7 299 347
- JP-A- 2003 225 599
- US-A- 5 497 232
- US-A- 5 589 225
- D.BONAMY ET AL.: 'Experimental study of granular surface flows via a fast camera:A continuous description' PHYSICS OF FLUIDS vol. 14, no. 5, May 2002, pages 1666 - 1673, XP008171398

## Description

### Technical Field

The present invention relates to a coating apparatus for performing coating, mixing, drying of medical, food, pesticidal products, and the like in a granular form, and more particularly, to a coating apparatus including a rotating drum to be driven to rotate about its axis, and a coating method using the coating apparatus.

### Background Art

Coating apparatus including a rotating drum (also referred to as "pan coating apparatus") have been used for performing film coating, sugar coating, and the like of medical, food, pesticidal products, and the like, which are prepared as tablets, soft capsules, pellets, grains, and in other similar forms (hereinafter collectively referred to as "particles").

For example, the following Patent Literatures 1 and 2 disclose this type of coating apparatus.

Patent Literature 1 discloses a coating apparatus including a ventilated-type rotating drum to be driven to rotate about a horizontal axis. The rotating drum includes a polygonal cylindrical peripheral wall portion, one end portion formed into a polygonal pyramid shape extending from one end of the peripheral wall portion in an axial direction toward one side, and another end portion formed into a polygonal pyramid shape extending from another end of the peripheral wall portion in the axial direction toward another side. A porous plate is mounted to each surface of the peripheral wall portion so that the peripheral wall portion is ventilated through porous portions of the porous plates. A jacket is mounted on an outer periphery of each porous plate, and a ventilation channel is formed between the jacket and the porous plate.

On the another end side of the rotating drum where a rotary drive mechanism including a motor and the like is installed, a ventilation mechanism is disposed for controlling the ventilation of process gas such as dry air for the rotating drum. The ventilation mechanism has a function of communicating, to an air inlet duct and to an air outlet duct, respectively, the ventilation channels that have reached a predetermined location in association with rotation of the rotating drum.

For example, when one of the ventilation channels reaches an upper portion of the rotating drum in association with the rotation of the rotating drum, the ventilation channel communicates to the air inlet duct, and when one of the ventilation channels reaches a lower portion of the rotating drum, the ventilation channel communicates to the air outlet duct. Thus, the process gas introduced from the air inlet duct in the ventilation channel at the upper portion of the rotating drum flows into the rotating drum through the porous plate at the upper portion of the peripheral wall portion, passes through a particle layer (rolling bed), flows out into the ventilation channel through the porous plate at the lower portion of the peripheral wall portion, and is exhausted into the air outlet duct through the ventilation channel.

Patent Literature 2 discloses a coating apparatus including a ventilated-type rotating drum which receives particles to be processed and is driven to rotate about an axis thereof. The rotating drum includes, along an axial direction thereof, one end portion, another end portion, and a peripheral wall portion connecting the one end portion and the another end portion to each other. The other end portion is positioned on a side of a rotary drive mechanism for driving and rotating the rotating drum. The one end portion and the other end portion are respectively provided with ventilation holes. The ventilation hole of one of the one end portion and the other end portion serves as an air inlet for supplying process gas from an outside to an inside of the rotating drum. The ventilation hole of another of the one end portion and the other end portion serves as an air outlet for exhausting the process gas from the inside to the outside of the rotating drum. The process gas supplied through the air inlet to the inside of the rotating drum passes through a particle layer in the rotating drum, and then is exhausted from the air outlet.

A rotating drum is a ventilated-type rotating drum, and the ventilation holes are provided at one end portion and another end portion. However, air passage portions (porous portions) for supply and exhaust of air are not provided in the peripheral wall portion. Accordingly, unlike the conventional ventilated-type rotating drum, it is unnecessary to provide a complex ventilation structure in which the air passage portions (porous portions) in the peripheral wall portion are covered with jackets from the outer periphery so that ventilation channels are formed. That is, the coating apparatus comprises such a ventilated-type rotating drum that does not comprise air passage portions (porous portions) for supply and exhaust of air in the peripheral wall portion of the rotating drum. In other words, the peripheral wall portion of the rotating drum has an air-tight structure, and ventilation channels formed by being covered with jackets are not provided on the outer periphery of the peripheral wall portion of the rotating drum. Thus, according to the coating apparatus, a cleaning operation and a validation operation after the cleaning operation can be performed more easily and more reliably than those for the conventional apparatus.

The ventilation hole of one of the one end portion and the another end portion is dedicated for use as an air inlet, and the ventilation hole of another of the one end portion and the another end portion is dedicated for use as an air outlet. Process gas (hot air, cool air, and the like) supplied into the rotating drum through the air inlet at the one end portion or the another end portion passes through the particle layer in the rotating drum, and then is exhausted from the air outlet at the another end portion or the one end portion. Thus, ventilation is distributed up to the inside of the particle layer, with the result that a process on the particle layer, such as drying, is evenly and sufficiently performed.

The rotating drum is disposed under a state in which an axis thereof forms a predetermined angle θ within a range of 0°≤θ≤90° with respect to a horizontal line. In other words, the rotating drum is disposed and operated in any selected one of the following states: the axis of the rotating drum is parallel to the horizontal line (θ=0°) ; the axis of the rotating drum is parallel to a vertical line (θ=90°); and the axis of the rotating drum is inclined with respect to the horizontal line (0°<θ<90°). It is preferred that the rotating drum be disposed under a state in which the axis thereof is inclined at the predetermined angle θ with respect to the horizontal line. In this case, the inclination angle θ of the axis is set so as to fall within a range of 20°≤θ<70°, preferably 30°<θ<45°, particularly preferably 30° or 40°.

The axis of the rotating drum is inclined at the predetermined angle θ with respect to the horizontal line, and hence the volume of the particles that can be processed in the rotating drum increases. Therefore, more particles are processed per cycle. As a result, the production efficiency is enhanced. Further, the rotating drum is rotated about the inclined axis, and hence the particles received in the rotating drum are flown under a state of being moved in both the rotational direction and the axial direction in accordance with rotation of the rotating drum. Thus, high stirring and mixing effects on the particle layer are achieved. For example, even when what is called a baffle (stirring blade) is not disposed in the rotating drum, the stirring and mixing effects can be sufficiently achieved. As a matter of course, when the baffle is used together, the stirring and mixing effects are enhanced. When the axis of the rotating drum is inclined, normally, a rear end portion of the rotating drum is positioned on a diagonally lower side.

In the above-mentioned coating apparatus, spray nozzles for spraying a spray liquid are arranged in the rotating drum, and the spray liquid is sprayed from the spray nozzles to the particle layer in the rotating drum. The spray liquid sprayed to the particle layer is spread over a surface of each particle by the stirring and mixing effects on the particle layer in accordance with the rotation of the rotating drum, and then is dried due to the process gas which passes through the particle layer. In this way, a coating film is formed on the surface of each particle in the particle layer.

Further, Patent Literature 3 describes a coating apparatus in which tablets under a coating process are imaged by using a CCD camera and a high speed stroboscopic apparatus so that, based on the obtained images, film thicknesses, surface conditions (smoothness and the like), presence/absence of aggregation, color unevenness, and the like of the tablets are evaluated, and an amount of a coating liquid, that is, the spray liquid, an r.p.m of the drum, an amount of air, and a temperature of hot air are controlled.

### Citation List

Patent Literature 1: JP 2001-58125 A
Patent Literature 2: JP 2004-148292 A
Patent Literature 3: JP 7-794 A

### Summary of

### Technical Problems

In a coating process on particle products (tablet and the like), which is performed by using the coating apparatus of this type, in order to secure satisfactory coating quality, it is necessary to perform a processing operation of uniformly spreading the spray liquid sprayed to the particle layer over the surface of each particle. For this processing operation, it is important to set operating conditions such as an r.p.mof the drum in accordance with a flow condition of the particle layer in the rotating drum.

Conventionally, the flow condition of the particle layer in the rotating drum is observed by an operator, and the operating conditions are determined based on experience of the operator. However, it is difficult to appropriately evaluate the flow condition of the particle layer by sight, which sometimes causes deterioration of the coating quality and production of defective products. Further, in the case of tablet coating and the like, depending on prescription, a lubricating effect is imparted to the tablets or frictional resistance of the tablets is enhanced in accordance with progress of the coating process. Thus, the flow condition of the tablet layer is successively changed. However, it is impossible for the operator to observe such subtle changes in movement in the particle layer in accordance with progress of the coating process. Thus, external appearances of the products tend to be deteriorated, and defective products tend to be produced.

It is therefore an object of the present invention to enhance coating quality and a yield of particle products by accurately detecting the flow condition of the particle layer in the rotating drum and controlling the operating conditions in accordance with the detection results.

### Solution to Problems

Through studies on the coating process on particle products, which is performed by using the coating apparatus of this type, the inventors of the present application have found that the flow condition of the particle layer can be accurately understood by detecting a flow rate of a particle in a surface layer portion of the particle layer in the rotating drum, and that coating quality and a yield of products can be enhanced by controlling operating conditions by using the flow rate of the particle as an index value. The present invention has been proposed based on this finding. Specifically, the present invention provides a coating apparatus, comprising: a rotating drum which receives particles to be processed and is driven to rotate about an axis of the rotating drum, the coating apparatus being configured to perform ventilation of process gas and spraying of a coating solution for a particle layer in the rotating drum so that a film is formed on a surface of each of the particles, the coating apparatus comprising: a high speed camera for imaging the particle layer in the rotating drum; an arithmetic processing apparatus for calculating a flow rate of the particle in the particle layer based on image data from the high speed camera; and a control apparatus for controlling, based on a calculation value obtained by the arithmetic processing apparatus, at least one operating condition selected from among a spraying condition of the coating solution, a ventilation condition of the process gas, and a rotational speed of the rotating drum. Here, the high speed camera is also referred to as a super motion camera, which generally comprises cameras capable of imaging at 30 frames or more per second. Specifically, in the market, cameras capable of imaging at approximately from 500 frames per second to 10,000 frames per second are dominant as the high speed camera. Cameras capable of imaging at 100,000 frames or more per second are sometimes referred to as an ultra high speed camera. The number of frames per second of the high speed camera used in the present invention is not particularly limited, and may be appropriately selected in consideration of a configuration of the coating apparatus, operating conditions, a price of the camera, and the like.

In the above-mentioned configuration, based on a plurality of image data pieces (frames) obtained from the high speed camera, the arithmetic processing apparatus calculates a moving distance (displacement amount) and a flow rate of a particular particle in a surface layer portion of the tablets, and outputs the flow rate to the control apparatus. The particular particle, which is subjected to calculations of the moving distance and the flow rate, may comprise a single particle or a plurality of particles. When the flow rate of each of the plurality of particles is calculated, a maximum value, a minimum value, or an average value of the flow rates thus calculated may be output to the control apparatus.

For example, when the rotating drum has a polygonal shape, or when a baffle is provided in the rotating drum, the stirring and mixing effects on the particle layer may vary depending on the position of the rotating drum in the rotational direction, and the flow condition of the particle layer may also vary depending on the position of the rotating drum in the rotational direction. In this case, the coating apparatus may have a configuration in which the arithmetic processing apparatus outputs the calculation value of the flow rate to the control apparatus when a reference position of the rotating drum reaches a predetermined position in a rotational direction of the rotating drum in accordance with rotation of the rotating drum and may control the operating condition based on the calculation value of the flow rate. With this, variation in measurement of the flow rate of the particle can be suppressed, and hence more accurate control can be performed.

The flow rate of the particle in the particle layer significantly depends on the rotational speed of the rotating drum, and hence it is preferred that the rotational speed of the rotating drum be selected as the at least one operating condition to be controlled.

In addition, in order to solve the above-mentioned problems, the present invention provides a coating method, comprising the steps of receiving particles to be processed in a rotating drum driven to rotate about an axis of the rotating drum, performing ventilation of process gas and spraying of a coating solution for a particle layer in the rotating drum so that a film is formed on a surface of each of the particles, imaging, by a high speed camera, the particle layer in the rotating drum, calculating a flow rate of the particle in the particle layer based on image data from the high speed camera, and controlling a rotational speed of the rotating drum based on a calculation value of the flow rate. Also in this invention, the step of controlling may comprise controlling the rotational speed of the rotating drum based on the calculation value of the flow rate at a time when a reference position of the rotating drum reaches a predetermined position in a rotational direction of the rotating drum in accordance with rotation of the rotating drum.

### Advantageous Effects of Invention

According to the present invention, the flow condition of the particle layer in the rotating drum can be accurately detected and the operating conditions are controlled based on the detection results. As a result, the coating quality and the yield of particle products are enhanced.

### Brief Description of Drawings

[FIG. 1] A schematic sectional view of a coating apparatus according to an embodiment of the present invention.
[FIG. 2] A schematic view of the coating apparatus according to the embodiment of the present invention.
[FIG. 3] A graph showing the embodiment of the present invention, in which a calculation value of a flow rate is output to a control apparatus in synchronization with rotation of a rotating drum.
[FIG. 4] A view illustrating a display example of image data (analysis moving image) from a high speed camera, a moving distance, and the flow rate.

### Description of Embodiments

In the following, description is made of an embodiment of the present invention.

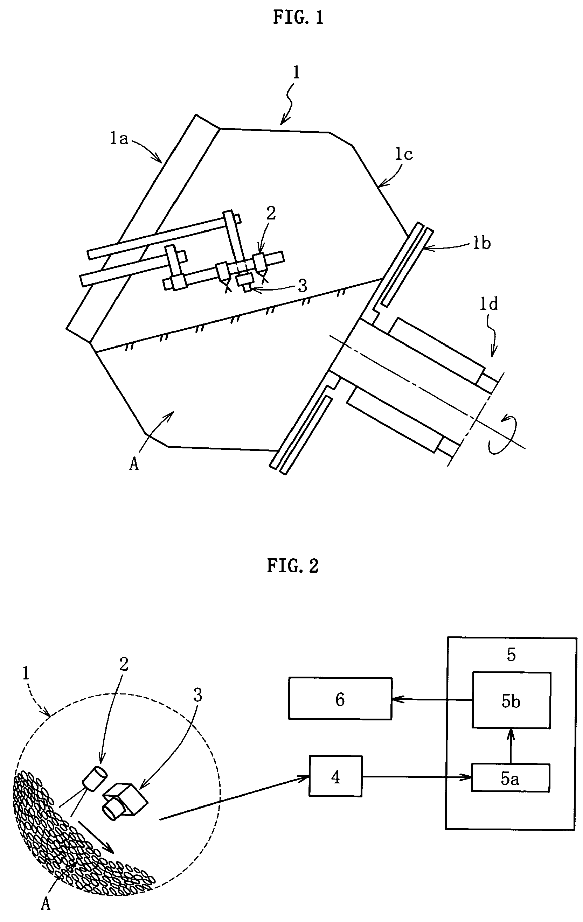

FIG. 1 conceptually illustrates a coating apparatus according to the embodiment of the present invention. The coating apparatus has the same basic structure as that of the coating apparatus described in JP 2004-148292 A (Patent Literature 2), and comprises a ventilated-type rotating drum 1 driven to rotate about an axis inclined with respect to a horizontal line.

The rotating drum 1 comprises, along an axial direction thereof, one end portion 1a, another end portion 1b, and a peripheral wall portion 1c of a polygonal cross section connecting the one end portion 1a and the another end portion 1b to each other. The another end portion 1b is positioned on a side of a rotary drive mechanism 1d for driving and rotating the rotating drum 1. The one end portion 1a and the other end portion 1b are respectively provided with ventilation holes. The ventilation hole of the one end portion 1a serves as an air inlet for supplying process gas from an outside to an inside of the rotating drum 1, and the ventilation hole of the another end portion 1b serves as an air outlet for exhausting the process gas from the inside to the outside of the rotating drum 1. The process gas (hot air or the like) supplied through the air inlet of the one end portion 1a to the inside of the rotating drum 1 passes through a particle layer A in the rotating drum 1, and then is exhausted from the air outlet of the another end portion 1b. Further, spray nozzles 2 for spraying a spray liquid (coating solution) are arranged in the rotating drum 1, and the spray liquid is sprayed from the spray nozzles 2 to the particle layer A in the rotating drum 1. The spray liquid sprayed to the particle layer A is spread over a surface of each particle due to stirring and mixing effects in accordance with rotation of the rotating drum 1, and then is dried by the process gas which passes through the particle layer A. In this embodiment, particles to be subjected to a coating process are tablets of medical products and the like. In the following, the particle layer A is also referred to as "tablet layer A."

In this embodiment, a high speed camera 3 is arranged in the rotating drum 1. The high speed camera 3 is arranged at a position at which the high speed camera 3 is capable of imaging a surface layer portion of the tablet layer A flowing due to the stirring and mixing effects in accordance with the rotation of the rotating drum 1. Thus, at predetermined time intervals, the high speed camera 3 successively images tablets in the surface layer portion of the tablet layer A, which roll and pass through a spray zone of the spray liquid sprayed from the spray nozzles 2.

The tablets received in the tilted rotating drum 1 flow in a state of being moved in both a rotational direction and an axial direction of the rotating drum 1 in accordance with the rotation of the rotating drum 1. The tablets flow mainly in the rotational direction. Specifically, as schematically illustrated in FIG. 2 (the peripheral wall portion 1c of the rotating drum 1, which actually has a polygonal shape, has a circular shape in illustration of FIG. 2), many of the tablets which are lifted forward in the rotational direction in accordance with the rotation of the rotating drum 1 flow so as to return forward in the rotational direction while rolling in the surface layer portion of the tablet layer A by their own weights. The high speed camera 3 images such a flow condition (rolling) of the tablets in the surface layer portion of the tablet layer A. Then, image data pieces of the tablet layer A, which is imaged by the high speed camera 3, are successively output to an arithmetic processing apparatus, for example, a personal computer 4. Based on the image data pieces, the personal computer 4 successively calculates a moving distance (displacement amount) and a flow rate of the tablet in the tablet layer A. Calculation values of the flow rate of the tablet, which are calculated by the personal computer 4, are successively input to a process determination portion 5a of a control apparatus 5. In the process determination portion 5a, there are registered in advance information pieces of tablets to be processed, such as types (tablet shape and the like), a preparation amount, film conditions (type of the coating solution, film thickness, and the like), and operating conditions (rotational speed of the rotating drum, spraying rate of the coating solution, ventilation condition, and the like) . Based on the calculation values of the flow rate of the tablet, which are successively input from the personal computer 4, and the above-mentioned various information pieces registered in advance, the process determination portion 5a outputs command signals to a drive control portion 5b so that the flow rate of the tablet becomes an optimum rate. Then, based on the command signals successively input from the process determination portion 5a, the drive control portion 5b successively outputs control signals to a rotary drive apparatus 6 of the rotating drum 1 so that the rotational speed of the rotating drum 1 is adjusted to an optimum speed.

For example, when a relationship between the tablet shape and the preparation amount of the tablets to be processed and a flow rate of a particular tablet, at which optimum coating quality is achieved, is experimentally determined in advance and registered in the process determination portion 5a of the control apparatus 5 (on a premise that the type of the coating solution, the spraying rate of the coating solution, the ventilation condition, and the like are unchanged), and when the rotational speed of the rotating drum 1 is successively controlled based on the image data pieces of the tablet layer A, which is imaged by the high speed camera 3, so that the flow rate of the particular tablet reaches a predetermined value, the flow condition of the tablet layer A is maintained to be optimum irrespective of the tablet shape and the preparation amount of the tablets. In this way, tablet products with satisfactory coating quality can be manufactured.

Further, when a relationship between the spraying rate of the coating solution and the flow rate of the particular tablet, at which a wetting amount of the tablets sprayed with the coating solution (appropriate amount of the coating solution to be sprayed per unit area of the tablet layer) becomes uniform, is experimentally determined in advance and registered in the process determination portion 5a of the control apparatus 5, and when the rotational speed of the rotating drum 1 is successively controlled based on the image data pieces of the tablet layer A, which is imaged by the high speed camera 3, so that the flow rate of the particular tablet reaches a predetermined value, the flow condition of the tablet layer A is maintained to be optimum even when the spraying rate of the coating solution is changed. As a result, the tablets are prevented from becoming excessively wet. Also in this way, tablet products with satisfactory coating quality can be manufactured.

Further, even when a scale of the coating apparatus is changed, by successively controlling the rotational speed of the rotating drum 1 so that the flow rate of the tablet in the tablet layer A is maintained, the same coating operation as with the original scale can be reproduced. Thus, variation of coating quality from scale to scale can be reduced. In other words, the flow rate of the tablet in the tablet layer A serves as a quantitative index value for setting the operating conditions when the scale is changed.

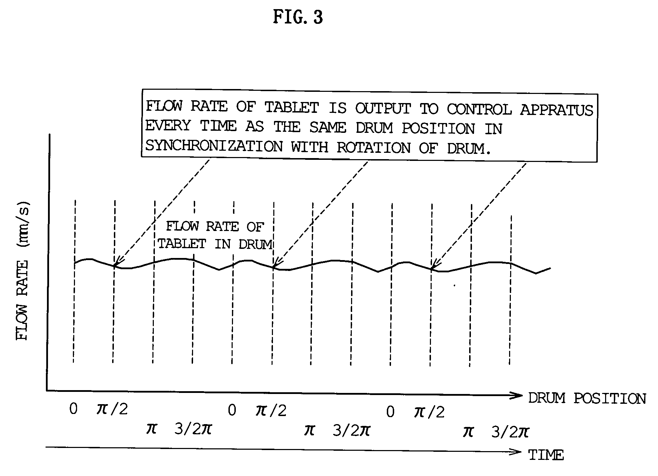

The rotating drum 1 has a polygonal shape, and in some cases, a baffle is provided at a predetermined position in the rotating drum 1. Thus, the stirring and mixing effects on the tablet layer A may vary depending on the position of the rotating drum 1 in the rotational direction, and the flow condition of the tablet layer A may also vary depending on the position of the rotating drum 1 in the rotational direction. In terms of this, as shown in FIG. 3, there may be employed a configuration in which, every time a reference position of the rotating drum 1 reaches a predetermined position in the rotational direction in accordance with the rotation of the rotating drum 1, the calculation value of the flow rate is output from the personal computer 4 to the control apparatus 5, and the rotational speed of the rotating drum 1 is controlled based on the calculation value of the flow rate. With this, variation in measurement of the flow rate of the tablet can be suppressed, and hence more accurate control can be performed. FIG. 3 shows an example of a configuration in which rotation of the rotary drive mechanism 1d of the rotating drum 1 is detected by rotation detecting means such as a rotary encoder, and the calculation value of the flow rate is output from the personal computer 4 to the control apparatus 5 every time the rotating drum 1 reaches a position (reference position) by being rotated in the rotational direction at 45° (n/2) with respect to a 0° position representing a position of the rotating drum 1 at a reference time point.

Further, the above-mentioned control based on the successive calculations of the flow rate of the tablet and the above-mentioned control based on the calculation of the flow rate of the tablet, which is performed at every predetermined timing in synchronization with the rotation of the rotating drum 1, may be switched with each other in accordance with variation of the calculation values of the flow rate. In other words, the above-mentioned control based on the successive calculations may be performed when the variation of the calculation values of the flow rate is small and the flow condition of the tablet layer A is stable, and the above-mentioned control based on the calculation at every predetermined timing may be performed when the variation of the calculation values of the flow rate is large.

Note that, the present invention is applicable not only to the coating apparatus configured as described above but also to the coating apparatus described in JP 2001-58125 A (Patent Literature 1) and coating apparatus in other configurations.

### Example 1

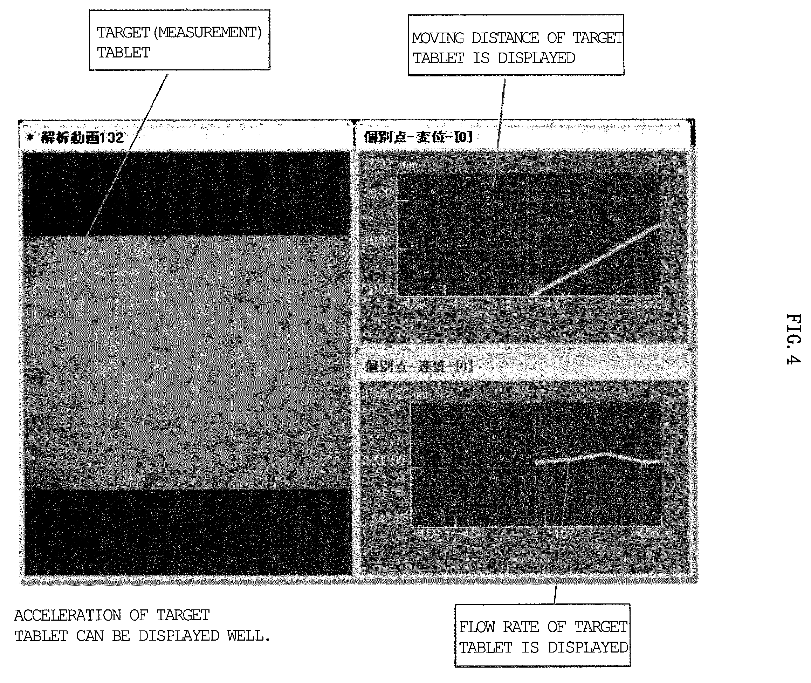

A predetermined amount of tablets was prepared into the rotating drum 1, and the tablet layer A was imaged with the high speed camera 3 while rotating the rotating drum 1 at a predetermined speed. Then, based on the image data pieces (moving images) from the high speed camera 3, the moving distance and the flow rate of the tablet in the tablet layer A were calculated with the personal computer 4. As the high speed camera 3, "VW-6000" manufactured by KEYENCE CORPORATION was used, and the tablet layer A was imaged at 90 fps. The image data pieces from the high speed camera 3 were input to the personal computer 4, and then subjected to an arithmetic process. In the personal computer 4, as illustrated in FIG. 4, a moving distance (mm) of one particular tablet (target tablet: target particle) was calculated based on the image data pieces of the surface layer portion of the tablet layer A. Then, a flow rate (mm/s) of the target tablet was calculated based on the moving distance per unit time . The moving distance of the target tablet can be calculated, for example, through an arithmetic operation using the personal computer 4 based on the number of pixels of the target tablet shown in a processed image of the image data pieces and an actual measured value of a size of the target tablet. Further, the target tablet can be automatically recognized with contrast between the tablet and voids therearound. The image data pieces (analysis moving images) from the high speed camera 3, the moving distance, and the flow rate can be displayed in a display of the personal computer 4 and also in a monitor screen of the control apparatus 5 as in the mode illustrated in FIG. 4. Note that, in addition to the flow rate (mm/s) of the target tablet, acceleration (mm/S²) thereof may be calculated and displayed.

### Reference Signs List

- 1: rotating drum
- 2: spray nozzle
- 3: high speed camera
- 4: personal computer
- 5: control apparatus

## Claims

1. A coating apparatus, comprising:
a rotating drum (1) which receives particles to be processed and is driven to rotate about an axis of the rotating drum, the coating apparatus being configured to perform ventilation of process gas and spraying of a coating solution for a particle layer in the rotating drum (1) so that a film is formed on a surface of each of the particles;
a high speed camera (3) for imaging the particle layer in the rotating drum;
an arithmetic processing apparatus (4) for calculating a flow rate of the particle in the particle layer based on image data from the high speed camera; and
a control apparatus (5) for controlling, based on a calculation value obtained by the arithmetic processing apparatus, at least one operating condition selected from among a spraying condition of the coating solution, a ventilation condition of the process gas, and a rotational speed of the rotating drum.

2. A coating apparatus according to claim 1, wherein the arithmetic processing apparatus outputs the calculation value of the flow rate to the control apparatus when a reference position of the rotating drum reaches a predetermined position in a rotational direction of the rotating drum in accordance with rotation of the rotating drum.

3. A coating apparatus according to claim 1 or 2, wherein the rotational speed of the rotating drum is selected as the at least one operating condition.

4. A coating method, comprising the steps of:
receiving particles to be processed in a rotating drum driven to rotate about an axis of the rotating drum;
performing ventilation of process gas and spraying of a coating solution for a particle layer in the rotating drum so that a film is formed on a surface of each of the particles;
imaging, by a high speed camera, the particle layer in the rotating drum;
calculating a flow rate of the particle in the particle layer based on image data from the high speed camera; and
controlling a rotational speed of the rotating drum based on a calculation value of the flow rate.

5. A coating method according to claim 4, wherein the step of controlling comprises controlling the rotational speed of the rotating drum based on the calculation value of the flow rate at a time when a reference position of the rotating drum reaches a predetermined position in a rotational direction of the rotating drum in accordance with rotation of the rotating drum.

## Patentansprüche

1. Beschichtungsvorrichtung, mit:
einer rotierenden Trommel (1), die zu bearbeitende Teilchen aufnimmt und angetrieben wird, um sich um eine Achse der rotierenden Trommel zu drehen,
wobei die Beschichtungsvorrichtung ausgebildet ist, um in der rotierenden Trommel (1) die Belüftung mit Prozessgas und das Aufsprühen einer Beschichtungslösung für eine Teilchenschicht durchzuführen, so dass auf einer Oberfläche jedes der Teilchen ein Film gebildet wird;
einer Hochgeschwindigkeitskamera (3), um die Teilchenschicht in der rotierenden Trommel abzubilden;
einer arithmetischen Verarbeitungsvorrichtung (4) zur Berechnung einer Flussrate des Teilchens in der Teilchenschicht basierend auf Bilddaten aus der Hochgeschwindigkeitskamera; und
einer Steuervorrichtung (5), um basierend auf einem Berechnungswert, der durch die arithmetische Verarbeitungsvorrichtung erzielt wurde, mindestens eine Betriebsbedingung zu steuern, die ausgewählt wird aus einer Sprühbedingung der Beschichtungslösung, einer Belüftungsbedingung des Prozessgases und einer Drehzahl der rotierenden Trommel.

2. Beschichtungsvorrichtung nach Anspruch 1, bei der die arithmetische Verarbeitungsvorrichtung den Berechnungswert der Flussrate an die Steuervorrichtung ausgibt, wenn eine Referenzposition der rotierenden Trommel in Übereinstimmung mit der Rotation der rotierenden Trommel eine vorherbestimmte Position in einer Drehrichtung der rotierenden Trommel erreicht.

3. Beschichtungsvorrichtung nach Anspruch 1 oder 2, bei der die Drehzahl der rotierenden Trommel als die mindestens eine Betriebsbedingung ausgewählt wird.

4. Beschichtungsverfahren, das folgende Schritte aufweist:
Aufnahme von zu verarbeitenden Teilchen in einer rotierenden Trommel, die angetrieben wird, um sich um eine Achse der rotierenden Trommel zu drehen;
Durchführung der Belüftung mit Prozessgas und des Aufsprühens einer Beschichtungslösung für eine Teilchenschicht in der rotierenden Trommel, so dass auf einer Oberfläche jedes der Teilchen ein Film gebildet wird,
Abbilden der Teilchenschicht in der rotierenden Trommel durch eine Hochgeschwindigkeitskamera;
Berechnung einer Flussrate des Teilchens in der Teilchenschicht basierend auf Bilddaten aus der Hochgeschwindigkeitskamera; und
Steuern einer Drehzahl der rotierenden Trommel basierend auf einem Berechnungswert der Flussrate.

5. Beschichtungsverfahren nach Anspruch 4, bei dem der Schritt der Steuerung die Steuerung der Drehzahl der rotierenden Trommel basierend auf dem Berechnungswert der Flussrate zu einem Zeitpunkt aufweist, an dem eine Referenzposition der rotierenden Trommel in Übereinstimmung mit der Rotation der rotierenden Trommel eine vorherbestimmte Position in einer Drehrichtung der rotierenden Trommel erreicht.

## Revendications

1. Appareil de revêtement comprenant :
- un tambour rotatif (1) qui reçoit des particules à traiter et est entraîné pour tourner autour d'un axe du tambour rotatif,
- l'appareil de revêtement étant configuré pour réaliser la ventilation du gaz de traitement et pulvériser une solution de revêtement pour une couche particulaire dans le tambour rotatif (1) de sorte qu'un film est formé sur une surface de chacune des particules ;
- une caméra à grande vitesse (3) pour imager la couche particulaire dans le tambour rotatif ;
- un appareil de traitement arithmétique (4) pour calculer un débit des particules dans la couche particulaire en fonction des données d'image provenant de la caméra à grande vitesse ; et
- un appareil de commande (5) pour commander, en fonction d'une valeur de calcul obtenue par l'appareil de traitement arithmétique, au moins une condition de fonctionnement choisie parmi une condition de pulvérisation de la solution de revêtement, une condition de ventilation du gaz de traitement et une vitesse de rotation du tambour rotatif.

2. Appareil de revêtement selon la revendication 1, dans lequel l'appareil de traitement arithmétique transmet la valeur de calcul du débit à l'appareil de commande lorsqu'une position de référence du tambour rotatif atteint une position prédéterminée dans une direction de rotation du tambour rotatif selon la rotation du tambour rotatif.

3. Appareil de revêtement selon la revendication 1 ou 2, dans lequel la vitesse de rotation du tambour rotatif est choisie comme étant la au moins une condition de fonctionnement.

4. Procédé de revêtement, comprenant les étapes consistant à :
- recevoir les particules à traiter dans un tambour rotatif entraîné pour tourner autour d'un axe du tambour rotatif ;
- réaliser la ventilation du gaz de traitement et pulvériser une solution de revêtement pour une couche particulaire dans le tambour rotatif de sorte qu'un film est formé sur une surface de chacune des particules ;
- imager, grâce à une caméra à grande vitesse, la couche particulaire dans le tambour rotatif ;
- calculer un débit des particules dans la couche particulaire en fonction des données d'image provenant de la caméra à grande vitesse ; et
- commander une vitesse de rotation du tambour rotatif en fonction d'une valeur de calcul du débit.

5. Procédé de revêtement selon la revendication 4, dans lequel l'étape de commande comprend l'étape consistant à commander la vitesse de rotation du tambour rotatif en fonction de la valeur de calcul du débit au moment où une position de référence du tambour rotatif atteint une position prédéterminée dans une direction de rotation du tambour rotatif selon la rotation du tambour rotatif.
